# EUROPÄISCHE PATENTANMELDUNG

(11) **EP 4 495 941 A1**
(43) Veröffentlichungstag der Anmeldung: **22.01.2025**
(21) Anmeldenummer: 23186781.3
(22) Anmeldetag: 20.07.2023
(51) Int. Cl.: G16H 30/20, G16H 40/63

(54) **MENSCH-MASCHINE-INTERAKTION ZUR BEDIENUNG EINER MEDIZINTECHNISCHEN VORRICHTUNG**

(71) Anmelder: Siemens Healthineers AG, 91301 Forchheim (DE)
(72) Erfinder: Alzaga, Amilcar, 96185 Schönbrunn im Steigerwald (DE)
(74) Vertreter: Siemens Healthineers Patent Attorneys

(57) **Zusammenfassung**

In einem Verfahren zur Mensch-Maschine-Interaktion zur Bedienung einer medizintechnischen Vorrichtung (1) wird eine erste Benutzereingabe erfasst wird, welche eine Absicht zur Bedienung der medizintechnischen Vorrichtung (1) für einen Anwendungsfall spezifiziert. Abhängig von der medizintechnischen Vorrichtung (1) und dem Anwendungsfall wird wenigstens ein Eingabevorschlag für ein computerimplementiertes Sprachmodell generiert und eine Darstellung des wenigstens einen Eingabevorschlags ausgegeben. Es wird eine zweite Benutzereingabe erfasst, welche einen ersten Eingabevorschlag des wenigstens einen Eingabevorschlags validiert oder den wenigstens einen Eingabevorschlag zurückweist. Falls der erste Eingabevorschlag durch die zweite Benutzereingabe validiert wurde, werden Benutzerinformationen zur Steuerung der medizintechnischen Vorrichtung (1) für den Anwendungsfall generiert und ausgegeben, indem das Sprachmodell auf den ersten Eingabevorschlag angewendet wird.

## Beschreibung

Die vorliegende Erfindung betrifft ein computerimplementiertes Verfahren zur Mensch-Maschine-Interaktion zur Bedienung einer medizintechnischen Vorrichtung, eine Datenverarbeitungsvorrichtung zur Durchführung eines solchen computerimplementierten Verfahrens sowie ein entsprechendes Computerprogrammprodukt.

Medizintechnische Vorrichtungen, beispielsweise Bildgebungsmodalitäten wie Ultraschallbildgebungssysteme, röntgenbasierte Bildgebungssysteme und Magnetresonanztomographiesysteme, oder Vorrichtungen zur Durchführung therapeutischer Verfahren, etwa HIFU-Systeme (HIFU steht für "Hochintensiver fokussierter Ultraschall") oder Histotripsie-Systeme, sind häufig sehr komplex und können eine große Anzahl von Funktionen aufweisen.

Um solche medizintechnischen Vorrichtungen adäquat nutzen zu können, ist häufig nicht nur eine medizinische Grundausbildung und eine Ausbildung spezifisch für die konkrete medizintechnische Vorrichtung erforderlich, sondern in hohem Maße auch Übung und praktische Anwendung. Zudem ist es gegebenenfalls erforderlich das jeweilige Wissen über die Arbeitsabläufe und Funktionen stets auf dem neuesten Stand zu halten.

In der Folge kann dies dazu führen, dass medizintechnische Vorrichtungen nicht korrekt bedient werden und/oder deren Funktionalität nicht ausgenutzt wird.

Sprachmodelle, insbesondere sogenannte große Sprachmodelle, LLMs (englisch: large language models), wie etwa das in der Veröffentlichung "GPT-4 Technical Report" (ar-Xiv:2303.08774v3) beschriebene GPT-4, können als virtuelle Assistenten genutzt werden. Ein Problem dabei sind irrelevante Aussagen oder sogenannte halluzinierte Antworten oder Aussagen, die durch die zugrundeliegenden Trainingsdaten nicht gerechtfertigt sind.

Es ist eine Aufgabe der vorliegenden Erfindung, ein Verfahren zur Mensch-Maschine-Interaktion zur Bedienung einer medizintechnischen Vorrichtung anzugeben, bei dem ein Benutzer zuverlässiger unterstützt werden kann.

Diese Aufgabe wird durch den Gegenstand des unabhängigen Anspruchs gelöst. Weiterführende Entwicklungen und bevorzugte Ausführungsformen sind Gegenstand der abhängigen Ansprüche.

Unabhängig vom grammatikalischen Geschlecht eines bestimmten Begriffes sind Personen mit männlicher, weiblicher oder anderer Geschlechteridentität mit umfasst.

Gemäß einem Aspekt der Erfindung wird ein computerimplementiertes Verfahren zur Mensch-Maschine-Interaktion zur Bedienung einer medizintechnischen Vorrichtung angegeben. Dabei wird eine erste Benutzereingabe erfasst, welche eine Absicht eines Benutzers zur Bedienung der medizintechnischen Vorrichtung für einen Anwendungsfall spezifiziert. Abhängig von der medizintechnischen Vorrichtung und dem Anwendungsfall wird wenigstens ein Eingabevorschlag für ein computerimplementiertes Sprachmodell generiert und eine Darstellung des wenigstens einen Eingabevorschlags wird an den ausgegeben. In Reaktion auf die Ausgabe der Darstellung des wenigstens einen Eingabevorschlags, also insbesondere nach der Ausgabe der Darstellung des wenigstens einen Eingabevorschlags, wird eine zweite Benutzereingabe erfasst, welche einen ersten Eingabevorschlag des wenigstens einen Eingabevorschlags validiert oder den wenigstens einen Eingabevorschlag zurückweist, also insbesondere alle Eingabevorschläge des wenigstens einen Eingabevorschlags zurückweist. Falls der erste Eingabevorschlag durch die zweite Benutzereingabe validiert wurde, werden Benutzerinformationen zur Steuerung der medizintechnischen Vorrichtung für den Anwendungsfall generiert und ausgegeben, indem das Sprachmodell auf den ersten Eingabevorschlag angewendet wird.

Sofern nicht anders angegeben, können alle Schritte des computerimplementierten Verfahrens von einer Datenverarbeitungsvorrichtung durchgeführt werden, welche wenigstens eine Recheneinheit aufweist. Insbesondere ist die wenigstens eine Recheneinheit zur Durchführung der Schritte des computerimplementierten Verfahrens konfiguriert oder angepasst. Hierzu kann die wenigstens eine Recheneinheit beispielsweise ein Computerprogramm speichern, das Befehle beinhaltet, die bei Ausführung durch die wenigstens eine Recheneinheit die wenigstens eine Recheneinheit dazu veranlassen, das computerimplementierte Verfahren auszuführen.

Eine medizintechnische Vorrichtung kann eine beliebige technische Vorrichtung zur, insbesondere wenigstens zum Teil automatischen, Durchführung eines diagnostischen, therapeutischen, chirurgischen und/oder bildgebenden Verfahrens sein. Die Durchführung eines diagnostischen, therapeutischen und/oder chirurgischen Verfahrens kann jedoch im Allgemeinen nicht als Bestandteil des erfindungsgemäßen computerimplementierten Verfahrens angesehen werden. Vielmehr können die erfindungsgemäß bereitgestellten Benutzerinformationen zur Durchführung eines solchen Verfahrens genutzt werden.

Bei dem computerimplementierten Sprachmodell handelt es sich insbesondere um ein sogenanntes großes Sprachmodell, LLM. Ein Eingabevorschlag kann als Vorschlag für eine Eingabe, auch als Prompt bezeichnet, in das Sprachmodell verstanden werden.

Das Sprachmodell kann insbesondere trainiert bereitgestellt werden, also ein bekanntes Sprachmodell sein. Es ist auch möglich, dass das Sprachmodell vortrainiert bereitgestellt wird und anhand weiterer Trainingsdaten verfeinert wird, beispielsweise über entsprechend bereitgestellte Schnittstelle zur Programmierung von Anwendungen, API (englisch: application programming interface).

Eine Benutzereingabe kann beispielsweise in Textform erfolgen oder auch in Sprachform, beispielsweise gefolgt von einer Sprache-zu-Text Umwandlung. Die Darstellung des wenigstens einen Eingabevorschlags beinhaltet eine Darstellung des ersten Eingabevorschlags und insbesondere, falls der wenigstens einen Eingabevorschlag zwei oder mehr Eingabevorschläge beinhaltet, jeweils eine Darstellung aller Eingabevorschläge des wenigstens einen Eingabevorschlags. Eine Darstellung eines Eingabevorschlags kann beispielsweise eine visuelle Darstellung sein, insbesondere eine Darstellung in Textform. Die Ausgabe der Darstellung des wenigstens einen Eingabevorschlags kann beispielsweise visuell auf einem Anzeigegerät erfolgen und/oder akustisch durch ein entsprechenden Lautsprechersystem.

Dass die zweite Benutzereingabe den ersten Eingabevorschlag validiert, kann derart verstanden werden, dass der Benutzer gemäß einem Inhalt der zweiten Benutzereingabe zum Ausdruck bringt, dass er dem ersten Eingabevorschlag zustimmt.

Dass die zweite Benutzereingabe den wenigstens einen Eingabevorschlag zurückweist, kann derart verstanden werden, dass der Benutzer gemäß einem Inhalt der zweiten Benutzereingabe zum Ausdruck bringt, dass er keinem Eingabevorschlag des wenigstens einen Eingabevorschlags zustimmt. Der Inhalt der zweiten Benutzereingabe kann sich darin erschöpfen oder weitere Informationen beinhalten, wie etwa eine Begründung der Zurückweisung oder einen Verbesserungsvorschlag und so weiter.

Die Ausgabe der Benutzerinformationen des wenigstens einen Eingabevorschlags kann beispielsweise visuell auf einem Anzeigegerät erfolgen und/oder akustisch durch das Lautsprechersystem.

Die erste Benutzereingabe spezifiziert beispielsweise die konkrete medizintechnische Vorrichtung und den konkreten Anwendungsfall für den der Benutzer die medizintechnische Vorrichtung anzuwenden wünscht. Die konkrete medizintechnische Vorrichtung kann beispielsweise aus einer Vielzahl vorgegebener medizintechnischer Vorrichtung gewählt werden. Darüber hinaus kann die erste Benutzereingabe in manchen Ausführungsformen auch weitere Informationen beinhalten, beispielsweise betreffend eine Ausbildung des Benutzers, einen Wissensstand des Benutzers, eine Zusammensetzung eines Teams von Menschen zur Durchführung der konkreten Anwendung, persönliche beziehungsweise anatomische oder pathologische Informationen eines Patienten, ein Krankheitsbild oder einen Krankheitsverlauf des Patienten, Ergebnisse früherer medizinischer Untersuchungen und so weiter.

Durch das mindestens zweistufige Vorgehen, nämlich die Bereitstellung des wenigstens einen Eingabevorschlags in Reaktion auf die erste Benutzereingabe und die Anwendung des Sprachmodells nur falls ein Eingabevorschlag validiert wird, können die Benutzerinformationen zuverlässiger bereitgestellt werden.

Gemäß zumindest einer Ausführungsform beinhalten die Benutzerinformationen Systemeinstellungen für die medizintechnische Vorrichtung.

Basierend auf den Benutzerinformationen kann der Benutzer also die medizintechnische Vorrichtung korrekt einstellen.

Gemäß zumindest einer Ausführungsform wird, falls der erste Eingabevorschlag durch die zweite Benutzereingabe validiert wurde, ein Parameter, beispielweise die Systemeinstellungen, der medizintechnischen Vorrichtung automatisch gemäß der Benutzerinformation eingestellt.

Gemäß zumindest einer Ausführungsform wird, falls der erste Eingabevorschlag durch die zweite Benutzereingabe validiert wurde, die medizintechnische Vorrichtung gemäß der Benutzerinformation automatisch angesteuert wird, um eine Messung automatisch durchzuführen.

Gemäß zumindest einer Ausführungsform beinhalten die Benutzerinformationen eine Anleitung zur Bedienung der medizintechnischen Vorrichtung für den Anwendungsfall, insbesondere in Textform.

Der Benutzer kann damit Schritt für Schritt angeleitet werden, um die notwendigen Bedienschritte vorzunehmen.

Gemäß zumindest einer Ausführungsform beinhalten die Benutzerinformationen eine oder mehrere Bilddarstellungen eines Vorgehens zur Bedienung der medizintechnischen Vorrichtung für den Anwendungsfall.

Der Benutzer kann dadurch visuell bei der Bedienung der medizintechnischen Vorrichtung unterstützt werden.

Gemäß zumindest einer Ausführungsform das beinhaltet Sprachmodell ein trainiertes künstliches neuronales Netzwerk.

Künstliche neuronale Netzwerke haben sich als überaus leistungsfähige Algorithmen insbesondere für große Sprachmodelle bewiesen.

Gemäß zumindest einer Ausführungsform ist das künstliche neuronale Netzwerk als generativer vortrainierter Transformer, GPT (englisch: generative pre-trained transformer) ausgestaltet.

Solche neuronalen Netzwerke, wie etwa das eingangs erwähnte GPT-4, haben sich als besonders leistungsfähig erwiesen.

Gemäß zumindest einer Ausführungsform beinhaltet der wenigstens eine Eingabevorschlag den ersten Eingabevorschlag und einen zweiten Eingabevorschlag. Durch die zweite Benutzereingabe wird entweder der erste Eingabevorschlag ausgewählt und validiert oder der wenigstens eine Eingabevorschlag zurückgewiesen wird, also insbesondere sowohl der erste Eingabevorschlag als auch der zweite Eingabevorschlag.

Der wenigstens eine Eingabevorschlag beinhaltet also zumindest zwei Eingabevorschläge, vorzugsweise drei oder mehr, aus denen der Benutzer wählen kann. So kann der Benutzer insbesondere denjenigen Eingabevorschlag auswählen, der seiner Intention am nächsten kommt. Auf diese Weise kann die Qualität der Benutzerinformationen verbessert werden beziehungsweise unnötige Wiederholungen oder Korrekturen können vermieden werden.

Gemäß zumindest einer Ausführungsform werden Quelleninformationen betreffend die Benutzerinformationen ausgegeben.

Die Quelleninformationen sind dabei insbesondere Informationen, welche eine oder mehrere Quellen angeben, basierend auf denen die Benutzerinformationen durch das Sprachmodell erzeugt wurden. Die Quellen sind dabei insbesondere Teil von Trainingsdaten, die zum Trainieren des Sprachmodells verwendet wurden.

Der Benutzer kann dadurch also die Benutzerinformationen also plausibilisieren oder verifizieren.

Gemäß zumindest einer Ausführungsform beinhalten die Quelleninformationen eine Text- und/oder Bilddarstellung wenigstens eines Auszugs einer Informationsquelle für die Benutzerinformationen und die Text- und/oder Bilddarstellung wird auf einem Anzeigegerät angezeigt wird, wobei ein für die Benutzerinformationen relevanter Teil visuell hervorgehoben wird.

Mit anderen Worten wird durch die Quelleninformationen nicht nur angegeben, auf welche Informationsquelle ein entsprechender Teil der Benutzerinformationen zurückgeht, sondern es wird konkret visuell hervorgehoben, welcher Teil, insbesondere welche Textpassage oder Textpassage und/oder welche Abbildung oder Abbildungen der Informationsquelle dem entsprechenden Teil der Benutzerinformationen zugrunde liegt. Dadurch wird die Plausibilisierung oder Verifizierung der Benutzerinformationen weiter vereinfacht.

Bei der Informationsquelle kann es sich beispielsweise um eine wissenschaftliche Veröffentlichung, ein Lehrbuch, eine Arbeitsanweisung und so weiter handeln.

Gemäß zumindest einer Ausführungsform wird, falls der wenigstens eine Eingabevorschlag durch die zweite Benutzereingabe zurückgewiesen wurde, abhängig von der medizintechnischen Vorrichtung und dem Anwendungsfall wenigstens ein weiterer Eingabevorschlag für das Sprachmodell generiert und eine Darstellung des wenigstens einen weiteren Eingabevorschlags ausgegeben. In Reaktion darauf wird eine dritte Benutzereingabe erfasst, welche einen ersten weiteren Eingabevorschlag des wenigstens einen weiteren Eingabevorschlags validiert oder den wenigstens einen weiteren Eingabevorschlag zurückweist. Falls der erste weitere Eingabevorschlag durch die dritte Benutzereingabe validiert wurde, weitere Benutzerinformationen zur Steuerung der medizintechnischen Vorrichtung für den Anwendungsfall generiert und ausgegeben werden, indem das Sprachmodell auf den ersten weiteren Eingabevorschlag angewendet wird.

So lässt sich die Qualität der Benutzerinformationen beziehungsweise der weiteren Benutzerinformationen weiter verbessern. Auf analoge Weise lässt sich iterativ vorgehen, bis ein validierter Eingabevorschlag vorliegt. Es ist jedoch auch möglich, dass das Verfahren abgebrochen wird, falls nach einer vordefinierten Anzahl von Iterationen kein validierter Eingabevorschlag vorliegt.

Gemäß zumindest einer Ausführungsform wird der wenigstens eine weitere Eingabevorschlag abhängig von einem Inhalt der zweiten Benutzereingabe generiert.

Mit anderen Worten beinhaltet die zweite Benutzereingabe nicht nur die Information, dass der wenigstens eine Eingabevorschlag zurückgewiesen wird, sondern weitergehende Informationen, wie aus Sicht des Benutzers ein verbesserter generiert erreichbar wäre. Diese weitergehenden Informationen können dann bei der Erzeugung des wenigstens einen weiterer Eingabevorschlags berücksichtigt werden.

Gemäß einer optionalen Ausgestaltung wird eine vierte Benutzereingabe erfasst. Die vierte Benutzereingabe wird insbesondere nach dem Generieren und/oder Ausgeben der Benutzerinformation erfasst. Die vierte Benutzereingabe spezifiziert eine Akzeptanz und/oder Verwertbarkeit der Benutzerinformationen für einen Anwendungsfall. Der Benutzer kann durch die vierte Benutzereingabe spezifizieren, ob die Benutzerinformation für den Anwendungsfall, den Patienten, die medizinische Vorrichtung brauchbar, anwendbar und/oder angemessen ist und/oder erscheint. Insbesondere kann der Benutzer mittels der vierten Benutzereingabe seine Zustimmung und/oder sein Misstrauen in die Benutzerinformation eingeben und/oder bereitstellen. Die Akzeptanz kann beispielsweise beschreiben, wie sehr der Benutzer in die Richtigkeit und/oder Angemessenheit der Benutzerinformation vertraut. Falls die vierte Benutzereingabe die Benutzerinformation als nicht akzeptabel, verwertbar, brauchbar, angemessen und/oder vertrauenswürdig spezifiziert, wird eine weitere, insbesondere verbesserte und/oder iterative, Benutzerinformation generiert und/oder ausgegeben. Die weitere, verbesserte und/oder iterative, Benutzerinformation wird durch Anwenden des Sprachmodells auf den ersten Eingabevorschlag und die vierte Benutzereingabe geniert und/oder bestimmt.

Gemäß zumindest einer Ausführungsform ist die medizintechnische Vorrichtung eine Bildgebungsmodalität, insbesondere ein Röntgenbildgebungssystem, ein Magnetresonanztomographiesystem oder ein ultraschallbasiertes Bildgebungssystem.

Für Anwendungsfälle oder Anwendungssituationen, die sich bei einem erfindungsgemäßen Verfahren ergeben können und die hierin nicht explizit beschrieben sind, kann vorgesehen sein, dass gemäß dem Verfahren eine Fehlermeldung und/oder eine Aufforderung zur Eingabe einer Nutzerrückmeldung ausgegeben und/oder eine Standardeinstellung und/oder ein vorbestimmter Initialzustand eingestellt wird.

Gemäß einem weiteren Aspekt der Erfindung wird eine Datenverarbeitungsvorrichtung aufweisend wenigstens eine Recheneinheit angegeben. Die wenigstens eine Recheneinheit ist dazu eingerichtet, ein erfindungsgemäßes computerimplementiertes Verfahren durchzuführen.

Unter einer Recheneinheit kann insbesondere ein Datenverarbeitungsgerät verstanden werden, das einen Verarbeitungsschaltkreis enthält. Die Recheneinheit kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "look-up table"), durchzuführen.

Die Recheneinheit kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "application-specific integrated circuit"), eines oder mehrere feldprogrammierbare Gate-Arrays, FPGA, und/oder eines oder mehrere Einchipsysteme, SoC (englisch: "system on a chip"). Die Recheneinheit kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere Digitalsignalprozessoren, DSP, enthalten. Die Recheneinheit kann auch einen physischen oder einen virtuellen Verbund von Computern oder sonstigen der genannten Einheiten beinhalten.

In verschiedenen Ausführungsbeispielen beinhaltet die Recheneinheit eine oder mehrere Hardware- und/oder Softwareschnittstellen und/oder eine oder mehrere Speichereinheiten.

Eine Speichereinheit kann als flüchtiger Datenspeicher, beispielsweise als dynamischer Speicher mit wahlfreiem Zugriff, DRAM (englisch: "dynamic random access memory") oder statischer Speicher mit wahlfreiem Zugriff, SRAM (englisch: "static random access memory"), oder als nicht-flüchtiger Datenspeicher, beispielsweise als Festwertspeicher, ROM (englisch: "read-only memory"), als programmierbarer Festwertspeicher, PROM (englisch: "programmable read-only memory"), als löschbarer programmierbarer Festwertspeicher, EPROM (englisch: "erasable programmable read-only memory"), als elektrisch löschbarer programmierbarer Festwertspeicher, EEPROM (englisch: "electrically erasable programmable read-only memory"), als Flash-Speicher oder Flash-EEPROM, als ferroelektrischer Speicher mit wahlfreiem Zugriff, FRAM (englisch: "ferroelectric random access memory"), als magnetoresistiver Speicher mit wahlfreiem Zugriff, MRAM (englisch: "magnetoresistive random access memory") oder als Phasenänderungsspeicher mit wahlfreiem Zugriff, PCRAM (englisch: "phase-change random access memory"), ausgestaltet sein.

Gemäß einem weiteren Aspekt der Erfindung wird ein Computerprogramm mit Befehlen angegeben. Bei Ausführung der Befehle durch eine Datenverarbeitungsvorrichtung, insbesondere eine erfindungsgemäße Datenverarbeitungsvorrichtung, veranlassen die Befehle die Datenverarbeitungsvorrichtung dazu, ein erfindungsgemäßes computerimplementiertes Verfahren durchzuführen.

Die Befehle können beispielsweise als Programmcode vorliegen. Der Programmcode kann beispielsweise als Binärcode oder Assembler und/oder als Quellcode einer Programmiersprache, zum Beispiel C, und/oder als Programmskript, zum Beispiel Python, bereitgestellt sein.

Gemäß einem weiteren Aspekt der Erfindung wird ein computerlesbares Speichermedium angegeben, das ein erfindungsgemäßes Computerprogramm speichert.

Das Computerprogramm und das computerlesbare Speichermedium sind jeweils als Computerprogrammprodukte mit den Befehlen.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren gezeigten Merkmale und Merkmalskombinationen können nicht nur in der jeweils angegebenen Kombination, sondern auch in anderen Kombinationen von der Erfindung umfasst sein. Es können insbesondere auch Ausführungen und Merkmalskombinationen von der Erfindung umfasst sein, die nicht alle Merkmale eines ursprünglich formulierten Anspruchs aufweisen. Es können darüber hinaus Ausführungen und Merkmalskombinationen von der Erfindung umfasst, die über die in den Rückbezügen der Ansprüche dargelegten Merkmalskombinationen hinausgehen oder von diesen abweichen.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

In den Figuren zeigen
- FIG 1: eine schematische Darstellung einer medizintechnischen Vorrichtung und einer beispielhaften Ausführungsform einer erfindungsgemäßen Datenverarbeitungsvorrichtung; und
- FIG 2: ein schematisches Ablaufdiagramm einer beispielhaften Ausführungsform eines erfindungsgemäßen computerimplementierten Verfahrens zur Mensch-Maschine-Interaktion zur Bedienung einer medizintechnischen Vorrichtung.

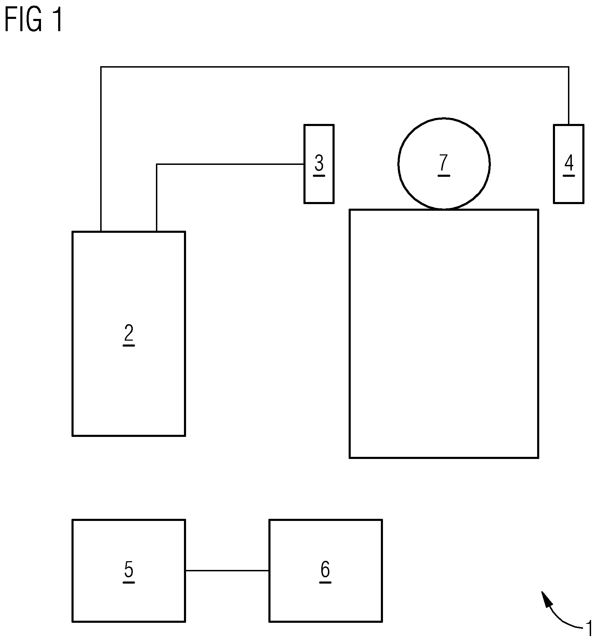

In FIG 1 sind eine medizintechnischen Vorrichtung 1 und eine beispielhafte Ausführungsform einer erfindungsgemäßen Datenverarbeitungsvorrichtung 5. Die Datenverarbeitungsvorrichtung 5 ist beispielsweise mit einem Anzeigegerät 6 verbunden.

Die medizintechnische Vorrichtung 1 ist rein beispielhaft und ohne Beschränkung der Allgemeinheit als Röntgenbildgebungssystem mit einer Röntgenquelle 3, einem Röntgendetektor 4 und einer Steuereinheit 2 dargestellt. Damit kann eine Röntgenbildgebung an einem Patienten 7 durchgeführt werden.

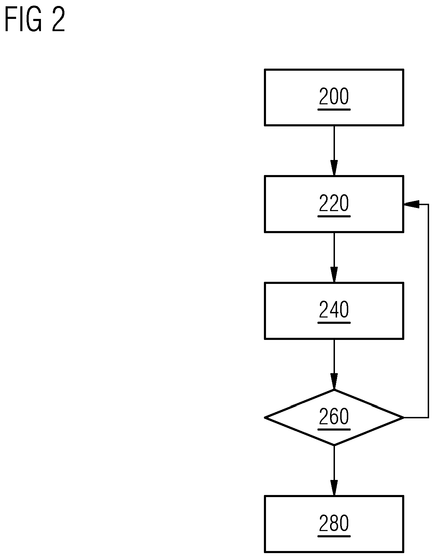

Die Datenverarbeitungsvorrichtung 5 weist wenigstens eine Recheneinheit auf, die dazu eingerichtet ist, ein erfindungsgemäßes computerimplementiertes Verfahren zur Mensch-Maschine-Interaktion zur Bedienung der medizintechnischen Vorrichtung 1 durchzuführen. Ein schematisches Ablaufdiagramm einer beispielhaften Ausführungsform eines solchen computerimplementierten Verfahrens ist in FIG 2 dargestellt.

In Schritt 200 wird eine erste Benutzereingabe erfasst, welche eine Absicht eines Benutzers zur Bedienung der medizintechnischen Vorrichtung 1 für einen bestimmten Anwendungsfall spezifiziert. In Schritt 220 wird abhängig von der medizintechnischen Vorrichtung 1 und dem Anwendungsfall wenigstens ein Eingabevorschlag für ein computerimplementiertes Sprachmodell generiert und eine Darstellung des wenigstens einen Eingabevorschlags, beispielsweise auf dem Anzeigegerät 6, ausgegeben.

In Schritt 240 wird eine zweite Benutzereingabe erfasst, welche einen ersten Eingabevorschlag des wenigstens einen Eingabevorschlags validiert oder den wenigstens einen Eingabevorschlag zurückweist. In Schritt 260 wird die zweite Benutzereingabe analysiert. Falls der wenigstens eine Eingabevorschlag durch die zweite Benutzereingabe zurückgewiesen wurde, wird das Verfahren beispielsweise mit Schritt 220 weitergeführt und es wird erneut wenigstens ein Eingabevorschlag generiert und so weiter.

Falls der erste Eingabevorschlag durch die zweite Benutzereingabe validiert wurde, werden in Schritt 280 Benutzerinformationen zur Steuerung der medizintechnischen Vorrichtung 1 für den Anwendungsfall generiert und, beispielsweise auf dem Anzeigegerät 6, ausgegeben, indem das Sprachmodell auf den ersten Eingabevorschlag angewendet wird. Das Sprachmodell kann dabei beispielsweise auf der wenigstens einen Recheneinheit und/oder einer anderen Recheneinheit gespeichert sein.

Als Sprachmodell kann in manchen Ausführungsformen beispielsweise das große Sprachmodell GPT-4 zugrunde gelegt werden, das mehrere Sprachen verarbeiten, Bilder erkennen und sehr umfangreiche Kontexteingabe erfassen kann. Es insbesondere mit verschiedenen Modalitäten wie Text, Bild, Video und Audio umgehen. Dieses Modell kann anhand spezifischer medizinischer Veröffentlichungen, beispielsweise über die medizinische Anwendung von Ultraschall, Röntgenstrahlung und so weiter, über Radiologie im Allgemeinen, über Differentialdiagnose oder Anamnese und so weiter, verfeinert werden.

In verschiedenen Ausführungsformen kann der Benutzer das System mittels natürlicher Sprache mit einem diagnostischen Verdacht beauftragen, woraufhin das System dem Benutzer verschiedene Möglichkeiten, möglicherweise auch mit alternativen medizintechnischen Vorrichtungen, anbieten kann und die bestmögliche Lösung im Rahmen der Möglichkeiten der medizintechnischen Vorrichtung 1 vorschlagen kann. Der Benutzer kann durch die Benutzerinformationen Schritt für Schritt angeleitet werden, zum Beispiel von der Positionierung des Patienten 7, über die Anordnung bestimmter Komponenten der medizintechnischen Vorrichtung 1, beispielsweise der Röntgenquelle 3 oder in anderen Ausführungsformen eines Ultraschallkopfes, bis zur Konfiguration der medizintechnischen Vorrichtung 1 und so weiter.

Da das System gewissermaßen weiß, wonach es suchen muss, könnte es in manchen Ausführungsformen auch automatisch Parameter der medizintechnischen Vorrichtung 1 einstellen, entsprechende Messungen vornehmen, und einen Bericht mit einer vorgeschlagenen Diagnose, in Betracht zu ziehenden Differenzialdiagnosen und/oder Folgeuntersuchungen zur Verfeinerung der Diagnose erstellen. Die Benutzerinformationen können mit kontextbezogenen Literaturhinweisen versehen werden, so dass der Benutzer die Vorschläge überprüfen kann.

Durch die Erfindung kann insbesondere das Risiko für halluzinierte Antworten des Sprachmodells reduziert werden.

Dazu kann das Verhalten des Sprachmodells insbesondere für bestimmte Anwendungsfelder im medizintechnischen Bereich angepasst werden, indem es mit einem für diese Anwendungsfelder relevanten Datensatz verfeinert wird. Dadurch kann das Wissen des Sprachmodells eingegrenzt werden, um die Wahrscheinlichkeit zu verringern, dass irrelevante oder halluzinierte Inhalte erzeugt werden.

Die Ausgaben des Sprachmodells können beispielsweise in einem Nachbearbeitungsschritt gefiltert werden, um Inhalte herauszufiltern, die bestimmte Kriterien nicht erfüllen, beispielsweise weil sie nicht aus vertrauenswürdigen Quellen stammen oder bestimmte Schlüsselwörter enthalten.

Ein Beispiel für einen Dialog des Benutzers mit dem System könnte folgt aussehen.

Benutzer: "Ich möchte Ultraschalluntersuchung nutzen, um eine Cholezystektomie zu diagnostizieren oder auszuschließen."

Eingabevorschlag: "Erläutere bitte ausführlich, wie ein Arzt ein bildgebendes Ultraschallverfahren zur Diagnose von Erkrankungen einsetzt, die eine Cholezystektomie erfordern könnten. Beginne mit einer Checkliste der benötigten Instrumente. Füge Informationen über das Ziel des Verfahrens, die beinhalteten Schritte und die Art und Weise, wie die Ultraschallergebnisse interpretiert werden, um die Notwendigkeit einer Cholezystektomie zu bestimmen, hinzu. Füge die Einstellungen für das Ultraschallsystem hinzu; Verwende nur Daten aus der validierten Bibliothek".

Nach der Validierung des Eingabevorschlags durch den Benutzer kann das Sprachmodell dann basierend darauf die Benutzerinformationen erzeugen.

Ein Beispiel für einen Eingabevorschlag und entsprechende Benutzerinformationen könnte wie folgt aussehen.

Eingabevorschlag: "Ich möchte, dass Sie als Radiologieexperte handeln und sich auf die Bildgebung der Leber konzentrieren. Da ich Kliniker bin, benötige ich keine Erklärungen Grundlagen. Ich werde einen klinischen Fall und einen diagnostischen Eindruck schildern und möchte, dass Sie mir die verfügbaren Optionen zur Bestätigung der Diagnose, mögliche Differentialdiagnosen und die für eine endgültige Diagnose in Frage kommenden Untersuchungen vorschlagen. Mein Fall ist: Ein 42-jähriger Patient, geboren in Mexiko, mit der persönlichen Vorgeschichte einer Appendektomie und einer Divertikelkrankheit mit etwa einer akuten Episode alle zwei Jahre in den letzten 8 Jahren. Der BMI liegt bei 27 und er trinkt etwa 3-4 Bier pro Woche und gelegentlich ein Glas Whiskey. Bei einer routinemäßigen Blutuntersuchung erschien der Lipasewert viermal über dem Schwellenwert (keine Bauchschmerzen), Transaminasen und Blutsenkungsgeschwindigkeit waren leicht erhöht. Blutbild, Nierenfunktion und Biochemie waren normal." Benutzerinformationen: "Aufgrund der Krankengeschichte und der Laborbefunde des Patienten gehe ich davon aus, dass ein Verdacht auf eine mögliche Lebererkrankung besteht. Hier sind einige Optionen für weitere Untersuchungen, Differentialdiagnosen und Behandlungen.

Diagnostische Optionen zur Bestätigung der Diagnose:
1. Ultraschall (US) - Ein Leber-Ultraschall ist ein nicht-invasives, kosteneffektives bildgebendes Verfahren der ersten Wahl, mit dem die Größe und Echotextur der Leber beurteilt und fokale Läsionen oder diffuse Anomalien identifiziert werden können.
2. Magnetresonanztomographie (MRT) - Die MRT mit hepatobiliärem Kontrastmittel oder leberspezifischen Kontrastmitteln kann detaillierte Informationen über das Leberparenchym und Läsionen liefern. Sie kann auch zur Beurteilung des Gallenbaums beitragen.
3. Computertomographie (CT) - Eine CT-Untersuchung mit intravenösem Kontrastmittel kann detaillierte Informationen über das Leberparenchym, die Gefäße und mögliche Läsionen liefern.

Mögliche Differentialdiagnosen:
1. Nichtalkoholische Fettlebererkrankung (NAFLD) - Angesichts des BMI des Patienten und der leicht erhöhten Transaminasen könnte eine NAFLD in Frage kommen. Diese kann in eine nichtalkoholische Steatohepatitis (NASH) und schließlich in eine Zirrhose übergehen.
2. Alkoholische Lebererkrankung - Auch wenn der Patient nur mäßig Alkohol konsumiert, könnte dies zu einer Lebererkrankung beitragen.
3. Hepatitis - Eine virale Hepatitis (A, B, C, D oder E), eine Autoimmunhepatitis oder eine medikamenteninduzierte Hepatitis sollten in Betracht gezogen werden.
4. Primär sklerosierende Cholangitis (PSC) - Die PSC ist eine seltene Lebererkrankung, die leicht erhöhte Transaminasen und Lipasen verursachen kann. Sie tritt häufiger bei Patienten mit entzündlichen Darmerkrankungen auf, kann aber auch bei Patienten mit Divertikelkrankheiten vorkommen.
5. Leberläsionen - Hämangiome, fokale noduläre Hyperplasie (FNH), hepatische Adenome oder hepatozelluläres Karzinom (HCC) können mögliche Ursachen für Leberanomalien sein.

Untersuchung zur Erlangung einer endgültigen Diagnose:
1. Serologie - Test auf virale Hepatitis-Marker (HAV, HBV, HCV, HDV, HEV), Autoimmunmarker (ANA, ASMA, LKM-1) und Leberfunktionstests (Albumin, Bilirubin, INR).
2. Bildgebung - Beginnen Sie mit einer Ultraschalluntersuchung der Leber, um strukturelle Anomalien festzustellen. Je nach Befund kann eine weitere Bildgebung mittels CT oder MRT angezeigt sein.
3. Elastographie - Mit FibroScan oder MR-Elastographie kann die Leberfibrose nichtinvasiv beurteilt und das weitere Vorgehen festgelegt werden.
4. Leberbiopsie - In Fällen, in denen die Diagnose ungewiss bleibt oder der Verdacht auf eine fortgeschrittene Lebererkrankung besteht, kann eine Leberbiopsie den endgültigen histologischen Nachweis erbringen.

Auf der Grundlage der Ergebnisse der Untersuchung kann eine endgültige Diagnose gestellt und eine angemessene Behandlung und Betreuung eingeleitet werden."

Es ist auch möglich, dass das System eine einzige umfassende Antwort bereitstellt, einschließlich beispielsweise der vorgeschlagenen Systemeinstellungen, der schrittweisen Vorgehensweise in Textform und schematischer Darstellungen jedes Schritts und einer entsprechenden klinischen Begründung. In manchen Ausführungsformen können visuelle Darstellungen der jeweiligen Informationsquellen, beispielsweise für jeden Absatz der Antwort, angezeigt werden, wobei der jeweilige Satz visuell hervorgehoben wird, so dass ein sachkundiger Benutzer ihn direkt validieren kann. Dies ist der Schlüssel, um die klinische Integration zu ermöglichen.

In manchen Ausführungsformen ist es auch möglich, dass medizinisches Personal ein AR-Headset trägt, über welches das System informieren kann, wie bestimmte Vorgänge durchgeführt werden sollen. Möglichweise hat das medizinische Personal dabei aber nicht die erforderlichen Qualifikationen, um die Anweisungen zu validieren. Die Validierung kann dann von einer entsprechend qualifizierten dritten Partei übernommen werden, die sich an einem anderen Ort befindet und beispielsweise mehrere Gruppen entsprechend betreut. Das Wissen und die Erfahrung der dritten Partei könnten dann ebenso effizient genutzt werden, wie die Ressourcen des medizinischen Personals und die Leistungsfähigkeit der entsprechenden Technologie.

Unabhängig vom grammatikalischen Geschlecht eines bestimmten Begriffes sind Personen mit männlicher, weiblicher oder anderer Geschlechteridentität mit umfasst.

## Patentansprüche

1. Computerimplementiertes Verfahren zur Mensch-Maschine-Interaktion zur Bedienung einer medizintechnischen Vorrichtung (1), wobei
- eine erste Benutzereingabe erfasst wird, welche eine Absicht zur Bedienung der medizintechnischen Vorrichtung (1) für einen Anwendungsfall spezifiziert;
- abhängig von der medizintechnischen Vorrichtung (1) und dem Anwendungsfall wenigstens ein Eingabevorschlag für ein computerimplementiertes Sprachmodell generiert und eine Darstellung des wenigstens einen Eingabevorschlags ausgegeben wird;
- eine zweite Benutzereingabe erfasst wird, welche einen ersten Eingabevorschlag des wenigstens einen Eingabevorschlags validiert oder den wenigstens einen Eingabevorschlag zurückweist; und
- falls der erste Eingabevorschlag durch die zweite Benutzereingabe validiert wurde, Benutzerinformationen zur Steuerung der medizintechnischen Vorrichtung (1) für den Anwendungsfall generiert und ausgegeben werden, indem das Sprachmodell auf den ersten Eingabevorschlag angewendet wird.

2. Computerimplementiertes Verfahren nach Anspruch 1, wobei die Benutzerinformationen
- Systemeinstellungen für die medizintechnische Vorrichtung (1) beinhalten; und/oder
- eine Anleitung zur Bedienung der medizintechnischen Vorrichtung (1) für den Anwendungsfall beinhalten; und/oder
- eine oder mehrere Bilddarstellungen eines Vorgehens zur Bedienung der medizintechnischen Vorrichtung (1) für den Anwendungsfall beinhalten.

3. Computerimplementiertes Verfahren nach einem der vorhergehenden Ansprüche, wobei das Sprachmodell ein trainiertes künstliches neuronales Netzwerk beinhaltet.

4. Computerimplementiertes Verfahren nach Anspruch 3, wobei das künstliche neuronale Netzwerk als generativer vortrainierter Transformer ausgestaltet ist.

5. Computerimplementiertes Verfahren nach einem der vorhergehenden Ansprüche, wobei der wenigstens eine Eingabevorschlag den ersten Eingabevorschlag und einen zweiten Eingabevorschlag beinhaltet und durch die zweite Benutzereingabe entweder der erste Eingabevorschlag ausgewählt und validiert wird oder der wenigstens eine Eingabevorschlag zurückgewiesen wird.

6. Computerimplementiertes Verfahren nach einem der vorhergehenden Ansprüche, wobei Quelleninformationen betreffend die Benutzerinformationen ausgegeben werden.

7. Computerimplementiertes Verfahren nach Anspruch 6, wobei die Quelleninformationen eine Text- und/oder Bilddarstellung wenigstens eines Auszugs einer Informationsquelle für die Benutzerinformationen beinhalten und die Text- und/oder Bilddarstellung auf einem Anzeigegerät (6) angezeigt wird, wobei ein für die Benutzerinformationen relevanter Teil visuell hervorgehoben wird.

8. Computerimplementiertes Verfahren nach einem der vorhergehenden Ansprüche, wobei, falls der wenigstens eine Eingabevorschlag durch die zweite Benutzereingabe zurückgewiesen wurde,
- abhängig von der medizintechnischen Vorrichtung (1) und dem Anwendungsfall wenigstens ein weiterer Eingabevorschlag für das Sprachmodell generiert und eine Darstellung des wenigstens einen weiteren Eingabevorschlags ausgegeben wird;
- eine dritte Benutzereingabe erfasst wird, welche einen ersten weiteren Eingabevorschlag des wenigstens einen weiteren Eingabevorschlags validiert oder den wenigstens einen weiteren Eingabevorschlag zurückweist; und
- falls der erste weitere Eingabevorschlag durch die dritte Benutzereingabe validiert wurde, weitere Benutzerinformationen zur Steuerung der medizintechnischen Vorrichtung (1) für den Anwendungsfall generiert und ausgegeben werden, indem das Sprachmodell auf den ersten weiteren Eingabevorschlag angewendet wird.

9. Computerimplementiertes Verfahren nach Anspruch 8, wobei der wenigstens eine weitere Eingabevorschlag abhängig von einem Inhalt der zweiten Benutzereingabe generiert wird.

10. Computerimplementiertes Verfahren nach einem der vorhergehenden Ansprüche, wobei eine vierte Benutzereingabe erfasst wird, welche eine Akzeptanz und/oder Verwertbarkeit der Benutzerinformationen für einen Anwendungsfall spezifiziert, wobei falls die Benutzerinformation durch die vierte Benutzereingabe als nicht akzeptiert und/oder nicht verwertbar spezifiziert wurde, generieren und ausgeben einer weiteren Benutzerinformationen zur Steuerung der medizintechnischen Vorrichtung (1) für den Anwendungsfall, indem das Sprachmodell auf den ersten Eingabevorschlag und die vierte Benutzereingabe angewendet wird.

11. Computerimplementiertes Verfahren nach einem der vorhergehenden Ansprüche, wobei die medizintechnische Vorrichtung (1) eine Bildgebungsmodalität ist.

12. Computerimplementiertes Verfahren nach einem der vorhergehenden Ansprüche, wobei, falls der erste Eingabevorschlag durch die zweite Benutzereingabe validiert wurde, ein Parameter der medizintechnischen Vorrichtung automatisch gemäß der Benutzerinformation eingestellt wird und/oder die medizintechnische Vorrichtung gemäß der Benutzerinformation automatisch angesteuert wird, um eine Messung automatisch durchzuführen.

13. Datenverarbeitungsvorrichtung (5) aufweisend wenigstens eine Recheneinheit, die zur Durchführung eines computerimplementierten Verfahrens einem der vorhergehenden Ansprüche eingerichtet ist.

14. Computerprogrammprodukt mit Befehlen, die, bei Ausführung durch eine Datenverarbeitungsvorrichtung (5) die Datenverarbeitungsvorrichtung (5) dazu veranlassen, ein computerimplementiertes Verfahren nach einem der Ansprüche 1 bis 12 durchzuführen.
